# EUROPEAN PATENT SPECIFICATION

(11) **EP 1 234 884 B1**
(45) Date of publication and mention of the grant of the patent: **20.08.2008**
(21) Application number: 01103845.2
(22) Date of filing: 16.02.2001
(51) Int. Cl.: C12P 9/00, C12P 17/06, C12P 7/26, C07F 7/18

(54) **A process for the preparation of beta-hydroxy-delta-lactone using novel intermediates**
Verfahren zur Herstellung eines Beta-Hydroxy-Delta-Lactons mittels neuer Zwischenprodukte
Procédé de préparation de béta-hydroxy-delta-lactone en utilisant des produits intermésiaires

(43) Date of publication of application: 28.08.2002
(73) Proprietor: Council of Scientific & Industrial Research, New Delhi 110001 (IN)
(72) Inventor: Sandeep, Raghunath Ghorpade, 008, Maharashtra (IN); Uttam, Ramrao Kalkote, 008, Maharashtra (IN); Subhash, Prataprao Chavan, 008, Maharashtra (IN); Sunil, Ramchandra Bhide, 008, Maharashtra (IN); Thottappillil, Ravindranathan, 008, Maharashtra (IN)
(74) Representative: Henkel, Feiler & Hänzel

(56) References cited:
- WO-A-93/06235
- WIRZ B ET AL: "Multiselective enzymatic reactions for the synthesis of protected homochiral cis- and trans-1,3,5-cyclohexanetriols" TETRAHEDRON: ASYMMETRY,ELSEVIER SCIENCE PUBLISHERS, AMSTERDAM,NL, vol. 11, no. 20, 20 October 2000 (2000-10-20), pages 4171-4178, XP004221070 ISSN: 0957-4166
- SUEMUNE H ET AL: "ASYMMETRIC HYDROLYSIS OF CIS,CIS-5-BENZYLOXY-1,3-DIACETOXY-CYCLOHEX ANE AND ITS APPLICATION TO THE SYNTHESIS OF CHIRAL LACTONE MOIETY IN COMPACTIN" TETRAHEDRON: ASYMMETRY,ELSEVIER SCIENCE PUBLISHERS, AMSTERDAM,NL, vol. 1, no. 7, 1990, pages 425-428, XP000986250 ISSN: 0957-4166

## Description

This invention relates to a **process for the preparation of optically active 6-hydroxymethyl-4-(*tert*-butyldimethylsilyloxy)-(4*S*,6*R*)-tetrahydro-2*H*-2-pyranone** (β Hydroxy - δ - Lactone) having formula **1**.

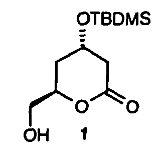

More particularly it relates to a process for the preparation of the said compound using compound having formula **5,** which was obtained from *cis, cis-3,5-* di(methylcarbo 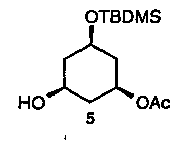 nyloxy)cyclohexylacetate having formula **2** by following the earlier patent procedure. 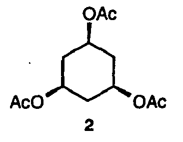 Hitherto known processes for the synthesis of β-hydroxy-δ-lactone (**1**) involves
a) Enzymatic kinetic resolution of racemic β-hydroxy-δ-lactone by transesterification with vinyl acetate in THF using *Chromobacteriun viscosum* lipase as catalyst at 40°C. [Crosby, J. B.; Andrew, J. H.; John, A. L. WO 9306235 A1 ***CA*** 119:93692 (1993)]
b) Chemoenzymatic route involving kinetic resolution through lactone formation in ether catalyzed by PPL [Bonini, C.; Pucci, P.; Viggiani, L. J. Org. Chem. 1991, 56, 4050]
c) Chemoenzymatic route involving enzymatic desymmetrization of intermediate diacetate, followed by chemical conversions. [Bonni, C.; Racioppi, R.; Righi, G.; Viggiani, L. J. Org. Chem. 1991, 58, 802]

The prior art processes have following drawbacks:
1. The processes use chemicals such as butyl lithium, lithium aluminum hydride, methoxydiethylborane that are costly and difficult to handle and therefore make the process difficult.
2. All known process are however involves large number of synthetic steps resulting in low over all yields.

The main object of the present invention is to provide a new process for the preparation of β-hydroxy-δ-lactone (**1**), which obviates the drawbacks of the prior art processes and use cheaper and easily accessible chemicals.

Another object of the present invention is to provide (i) selective Baeyer-Villiger rearrangement of 3-hydroxy-5-tert-butyldimethylsilyloxy-1-cyclohexanone (**7**) with chemical reagent or Baeyer Villiger oxidase and (ii) enantioselective hydrolysis of *cis*-3-(methylcarbonyloxy)-5-(*tert*-butyldimethylsilyloxy)cyclohexylacetate with enzyme. (iii) mild enzymatic hydrolysis of 3-oxo-5-(*tert*.butyldimethylsilyloxy)-(1*S*, 5*S*)-cyclohexylacetate (**6**) wherein β-elimination is suppressed and optical purity is enhanced through kinetic resolution.

Accordingly the present invention provides a process for the preparation of 6-hydroxymethyl-4- (tert.-butyldimethylsilyloxy)-(4S, 6R)-tetrahydro-2H-2-pyranone having formula 1 which comprises
a) reacting 3-hydroxy-5-(*tert*.butyldimethylsilyloxy)-(*1S*,*3R*,*5R*)-cyclohexylacetate having formula 5 in an organic solvent preferably chloroalkanes with chlorochromate of tertiary amines at temperature ranging from 10 to 30°C, quenching the reaction by adding of diethyl ether, filtering the mixture through ceilite and washing with brine, removing the solvent by evaporation, followed by fast column filtration to obtain 3-oxo-5-(*tert*.butyldimethylsilyloxy)-(1*S*, *5S*)-cyclohexylacetate having formula 6 , 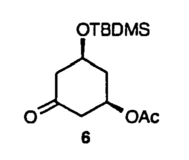
b) hydrolysing 3-oxo-5-(*tert*.butyldimethylsilyloxy)-(1*S*,5*S*)-cyclohexylacetate having formula 6 with lipase enzyme in a buffer having pH range of 5 to 7, at a temperature ranging from 25 to 30 °C for a period ranging between 24 to 48 hr, extracting the mixture with an organic solvent, removing the solvent by evaporation and on column chromatography to obtain 3-hydroxy-5-(*tert-*butyldimethyl silyloxy)-(*3R*,*5S*)-cyclohexan-1-one having formula 7, 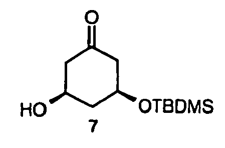
c) reacting 3-hydroxy-5-(*tert*-butyldimethylsilyloxy)-(3*R*,5*S*)-cyclohexan-1-one having formula **7** with m-chloroperbenzoic acid at room temperature for the period ranging from 16-24 hours, extracting with organic layer, washing with sodium metabisulphite, brine, drying and on evaporation to obtain 6-hydroxymethyl-4-(*tert*-butyldimethylsilyloxy)-(4*S*,6*R*)-tetrahydro-2*H*-2-pyranone having formula **1,**

In another embodiment of the present invention the organic solvent used in steps **a-c** for the extraction of the product is selected from the group consisting of ethyl acetate, chloroform and dichloromethane.

In another embodiment of the present invention the buffer used in steps **b** for the reaction is selected from phosphate buffer and citrate buffer.

In another embodiment of the present invention the lipase used in steps **b** for the reaction is selected from the group consisting of pig procain lipase (PPL), pig liver esterase (PLE) and chicken liver acetone powder (CLAP).

In yet another embodiment of the present invention the enzymatic hydrolysis used in step b is mild and suppresses B-elimination along with enhancement in optical purity.

In yet another embodiment the oxidising agent used is selected from the group consisting of chloroperbenzoic acid, hydrogen peroxide and peracetic acid.

In yet another embodiment the present invention provides a compound 3- oxo-5-(tert.butyldimethylsilyloxy-(1S,5S)-cyclohexylacetate having formula 6

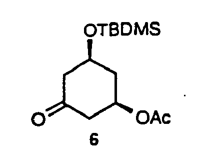

In yet another embodiment the present invention provides a compound 3-hydroxy-5-(tert-butyldimethyl silyloxy)-(3R,5S)-cyclohexane-1-one having formula 7

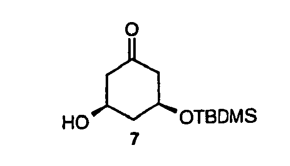

The process of the present invention is described herein below with references to the following examples, which are illustrative only and should not be construed to limit the scope of the present invention in any manner.

The novel components having formulae 3 to 5 have been claimed in our co-pending application EP-A-1 234 885

### Example 1

### Preparation of cis, cis-3-hydroxy-5-methylcarbonyloxy-cyclohexylacetate (3) :

Finely powdered *cis*, *cis*-3,5-di(methylcarbonyloxy)cyclohexylacetate(2,6.5 parts, 25.19 mmol parts) was suspended in 0.1 M sodium phosphate buffer (pH 7) (135 parts) and stirred vigorously. To the stirred suspension Porcine Liver Esterase (0.110 parts) was added and reaction mixture was stirred vigorously at 30°C for 20 hr. pH of the reaction mixture was monitored at every 2 hrs and was maintained at pH 7 using 1N NaOH solution. After completion of reaction (20 hr), it was extracted with ethyl acetate (2 x 150 parts). Organic layers were combined and washed with brine, dried on anhydrous sodium sulfate and concentrated under vacuum to yield *cis,cis*-3-hydroxy-5-methylcarbonyloxycyclohexylacetate (**3** yield (4.8 g parts, 88%).
1H NMR(CDCl₃): δ 1.20-1.58 (qn, 3H), 2.08 (s, 6H), 2.28 (m, 3H), 3.78 (m, 1H), 4.78 (m, 2H)
13C NMR (CDCl₃): δ 21.15, 36.33, 39.93, 64.83, 67.65, 170.42
IR (KBr): 754.60, 884.15, 1029.29, 1140.34, 1250.00, 1367.64, 1738.92, 2871.17, 2953.14, 3445.47
Mass: Base m/e = 96 other m/e: 156, 138, 114, 73, 67, 67, 60, 55
Elemental analysis: calculated for C₁₀H₁₆O₅: C 55.56%, H 7.40%
Found : C 55.30%, H 8.00%

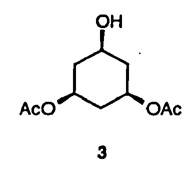

### Example 2

### Preparation of cis,cis-3-(methylcarbonyloxy)-5-(tert.butyldimethylsilyloxy)cyclohexylacetate (4)

*Cis, cis*-3-hydroxy-5-methylcarbonyloxy-cyclohexylacetate (**3**, 2 parts, 9.26 mmol) and DMAP (0.113 parts, 0.926 mmol) were placed in 100 ml two-necked round bottom flask equipped with dropping funnel and two-way stopcock. It was evacuated and flushed with argon. To it, dry dichloromethane (10 parts) and dry HMPA (2 part) was added and stirred to dissolve. The solution was cooled to -10°C with stirring. To it, solution of *tert*-butyldimethylsilyl chloride in 10 part dry dichloromethane was added dropwise while maintaining temperature below 0°C. Reaction mixture was stirred for 15 min and to it dry triethylamine (2.02 parts, 20 mmol) was added dropwise. Reaction mixture was stirred at room temperature for 12 hr. It was then transferred to a separating funnel and washed successively with cold, dil. HCl water, aq. NaHCO₃ and then brine. Organic layer was dried on anhydrous sodium sulfate and solvent was removed under vacuum. Residue was purified by flash column chromatography. (eluent 2-4% ethyl acetate in petroleum ether) to yield *Cis,cis*-3-(methylcarbonyloxy)-5-(*tert*.butyldimethylsilyloxy)cyclohexylacetate (**4**, yield 2.85 parts, 90%).
¹H NMR (CDCl₃): δ 0.06(s, 9H), 0.87 (s, 6H), 1.20-1.45 (m, 3H), 2.03 (s, 6H), 2.2 (m, 3H), 3.38 (m, 1H), 4.73 (m, 2H).
¹³C NMR (CDCl₃): δ -5.07, 17.61, 25.40, 36.17, 40.40, 65.43, 67.01, 169.64
IR (CHCl₃): 758.90, 838.05, 1034.91, 1106.32, 1246.82, 1368.91, 1734.01, 2858.81, 2955.07
Mass: Base m/e = 117 other m/e: 273, 213, 171, 159, 129, 117, 97, 79, 75, 57
Elemental analysis: calculated for C₂₆H₃₀O₅Si : C 58.185%, H 9.10%
Found : C 58.19%, H 9.50%

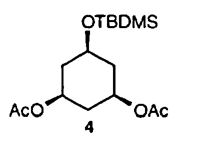

### Example 3

### Preparation of 3-hydroxy-5-(tert.butyldimethylsilyloxy)-(1S,3R,5R)-cyclohexyl acetate (5)

*Cis,cis*-3-(methylcarbonyloxy)-5-(*tert*.butyldimethylsilyloxy)cyclohexylacetate (**4**, 5 parts, 147 mmol) was dissolved in *tert*-butanol (20 parts). To the solution, 0.1 M sodium phosphate buffer (230 parts, pH 8) was added and mixture was stirred vigorously. To the stirred emulsion, Porcine liver esterase (0.150 parts) was added and the mixture was stirred vigorously at 30°C for 54 hrs. During reaction pH was maintained at 8 using 1N sodium hydroxide solution. Reaction mixture was extracted with ethyl acetate (3 x 200 parts). Organic layers were combined and washed with brine. It was then dried on anhydrous sodium sulfate and solvent was removed under vacuum. Oily residue contained 3-hydroxy-5-(*tert*.butyldimethylsilyloxy)-(1S,3*R*,5*R*)-cyclohexylacetate (**5**) along with unreacted (**4.**) Both were separated by flash column chromatography.
Cis,cis-3-(methylcarbonyloxy)-5-(*tert*.butyldimethylsilyloxy)cyclohexylacetate: (4, 1.07 parts);
3-hydroxy-5-(*tert*.butyldimethylsilyloxy)-(1*S*,3*R*,5*R*)-cyclohexylacetate: (**5**, 2.8 parts, 70% based on recovered starting material).
¹H NMR (CDCl₃) : δ 0.06 (s, 9H), 0.87 (s, 6H), 1.35-1.60, (m, 3H), 2.06 (s, 3H), 2.15 (m, 3H), 3.7 (m, 2H), 4.75 (m, 1H)
¹³C NMR (CDCl₃): δ -4.60, 18.15, 21.38, 25.90, 39.98, 40.31, 43.93, 65.45, 66.40, 68.17, 170.68
IR (CHCl₃): 758.43, 838.93, 1049.42, 1109.15, 1218.09, 1254.01, 1370.09, 1725.03, 2859.8, 2887.95, 2952.33, 3017.48
Mass: Base m/e = 75 other m/e: 231, 171, 129, 117, 105, 97, 79, 75, 67, 59
Elemental analysis: calculated for C₁₄H₂₈O₄Si : C 58.33%, H 9.72%
Found : C 58.15%, H 10.20%
Specific rotation [α]_{D} = - 4.8 (c 1, CHCl₃) e.e. >95%

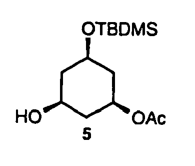

### Example 4:

### Preparation of 3-oxo-5-(tert.butyldimethylsilyloxy)-(1S, 5S)-cyclohexylacetate (6)

*Cis,cis*-3-(methylcarbonyloxy)-5-(*tert*.butyldimethylsilyloxy)-( 1*S*,3*R*,5*R*)-cyclohexylacetate (**5**, 1.40 parts, 2.78 mmol) was dissolved in dichloromethane 5 parts to the solution sodium acetate (0.1 part) and pyridinium chlorochromate (1.57 parts, 7.3 mmol) were added. The mixture was stirred for 5 hr at RT. Residue was extracted with ether (3x10 parts). Organic extracts were combined, filtered through ceilite. Filtrate was washed with brine:water (1:1), finally with brine. Organic layer was dried on anhydrous sodium sulphate and concentrated under vacuum. Residue was filtered through silica gel column to afford 3-oxo-5-(*tert*.butyldimethylsilyloxy)-(1*S*, 5*S*)-cyclohexylacetate (**6**, (1.30 parts, 93.5%).
1H NMR (CDCl₃): δ 0.10 (d, 9H), 0.88 (d, 6H), 2.06 (s, 3H), 2.45 (m, 4H), 2.70 (m,2H), 4.00 (m, 1H), 5.00 (m, 1H)
13C NMR (CDCl₃): δ -4.95, 17.82, 20.97, 25.57, 39.34, 45.93, 50.30, 65.98, 67.23, 169.87, 205.27
IR (CHCl₃): 442.57, 756.83, 1218.50, 1244.99, 1723.00, 2857.44, 2932.80, 2953.64, 3019.98
Mass: Base m/e = 163 other m/e: 185, 169, 145, 127, 117, 111, 101, 95, 75, 59
Elemental analysis: calculated for C₁₄H₂₆O₄Si : C 58.74%, 9.09%
Found : C 58.60%, H 9.8%
Specific rotation [α]_{D} = -11.54 (c 1, CHCl₃)

### Example 5

### Preparation of 3-hydroxy-5-(tert-butyldimethylsilyloxy)-(3R,5S)-cyclohexan-1-one (7)

3-Oxo-5-(*tert*.butyldimethylsilyloxy)-(1*S*, 5*S*)-cyclohexylacetate (**6**, 1.2 parts, 4.2 mmol) was dissolved in DMSO (10 parts) and 0.1 M phosphate buffer (pH 6.5, 90 parts). To the mixture, PLE (0.1 parts) was added and the reaction mixture was stirred for 48 hr at room temarature. It was filtered through ceilite and filtrate was extracted with ethyl acetate (3x100 parts). Organic extracts were combined and washed with brine. Organic layer was dried on anhydrous sodium sulphate and concentrated under vacuum. Residue was chromatographed on silica gel to afford 3-hydroxy-5-(*tert*-butyldimethylsilyloxy)-(3*R*,5*S*)-cyclohexan-1-one (**7** (0.77 parts, 75%).
1H NMR (CDCl₃): δ 0.08 (s, 6H), 0.85 (s, 9H), 1.95-2.4 (m, 2H), 2.45-2.78 (m, 4H), 3.96 (bs, 1H), 4.37 (m, 1H), 4.56 (m, 1H)
13C NMR (CDCl₃): δ -5.28, -5.50, 17.55, 25.34, 38.24, 49.56, 49.89, 68.86, 70.48, 206.78
IR (CHCl₃): 668.15, 759.18, 837.70, 1216.14, 1255.02, 1676.48, 1712.47, 2930.89, 2955.00, 3018.43, 3387.52, 3406.91
Mass: Base m/e = 75 other m/e: 187, 169, 145, 129, 101, 95, 75, 69, 59
Elemental analysis: calculated for C₁₂H₂₄O₃Si : C 59.01%, H 9.83%
Found : C 59.1%, H 9.98%
Specific rotation [α]_{D} = -22.06 (c 1, CHCl₃)

### Example 6

### Preparation of 6-hydroxymethyl-4-(tert-butyldimethylsilyloxy)-(4S,6R)-tetrahydro -2H-2-pyranone (1):

3-Hydroxy-5-(*tert*-butyldimethylsilyloxy)-(3*R*,5*S*)-cyclohexan-1-one (**7**, 0.1 part, 0.394 mmol) and 50% 3-chloroperbenzoic acid (0.275 parts, 0.79 mmol) were mixed and kept in dark for 24 hr. Reaction mixture was dissolved in ethyl acetate and washed successively with sodium metabisulfite solution, sodium bicarbonate solution followed by brine wash. It was then dried on anhydrous sodium sulfate and solvent was removed under vacuum. Residue was purified by flash column chromatography to yield white, crystalline 6-hydroxymethyl-4-(*tert*-butyldimethylsilyloxy)-(4*S*,6*R*)-tetrahydro-2*H*-2-pyranone (**1**, yield 0.035 part, 50%).
1H NMR (CDCl₃): δ 0.09, 0.07 (2s, 6H), 0.89 (s, 9H), 1.55-2.00(m + bs, 2H), 2.60
(d, 2H, J = 3), 3.66 (dd, 1H, J = 3, 12), 3.90 (dd, 1H, J = 3, 12), 4.38 (m, 1H), 4.80 (m, 1H)
13C NMR (CDCl₃): δ -5.17, 17.66, 25.41, 32.59, 39.90, 63.24, 64.19, 76.73, 170.1
IR (CHCl₃): 666.31, 898.12, 1021.88, 1061.06, 1086.17, 1118.98, 1390.84, 1463.17, 1729.43, 2857.28, 3418.25
Mass: Base m/e = 101 other m/e: 261, 229, 203, 185, 161, 143, 129, 111, 75, 68, 59
Elemental analysis: calculated for C₁₂H₂₄O₄Si: C 55.38%, H 9.20%
Found : C 55.40%, H 9.30%

Specific rotation [α]_{D} = -1.96 (c 1, CHCl₃) e.e. 99% (determined by chiral HPLC of corresponding benzoate derivative, column-Whelk-Ol [4.0 mm Id x 25 cm] AT-256; λ =254 nm, flow rate: 1 ml/min; mobile phase: hexane:isopropanol 92:08; retention time for benzoate of **1** = 20.10, for benzoate of **ent-1** = 17.48).

## Claims

1. A process for the preparation of 6-hydroxymethyl-4-(tert.-butyldimethylsilyloxy)-(4S,6R)-tetrahydro-2H-2-pyranone having formula 1 which comprises
a) reacting 3-hydroxy-5-(*tert*.butyldimethylsilyloxy)-(*1S*,3*R*,5*R*)-cyclohexylacetate having formula 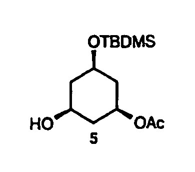 with chlorochromate of tertiary amines at temperature ranging from 10 to 30 °C, quenching the reaction by adding of diethyl ether, filtering the mixture through ceilite and washing with brine, removing the solvent by evaporation, followed by fast column filtration to obtain 3-oxo-5-(*tert*.butyldimethylsilyloxy)-(1*S*,5*S*)-cyclohexylacetate having formula 6, 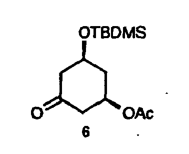
b) hydrolysing 3-oxo-5-(*tert*.butyldimethylsilyloxy)-(*1S*,5S)-cyclohexylacetate having formula 6 with lipase enzyme in a buffer having pH range of 5 to 7, at a temperature ranging from 25 to 30 °C for a period ranging between 24 to 48 hr, extracting the mixture with an organic solvent, removing the solvent by evaporation and on column chromatography to obtain 3-hydroxy-5-(*tert-*butyldimethyl silyloxy)-(*3R*,*5S*)-cyclohexan-1-one having formula 7, 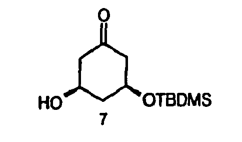
c) reacting 3-hydroxy-5-(*tert*-butyldimethyl silyloxy)-(3*R*,5*S*)-cyclohexan-1-one having formula 7 with m-chloroperbenzoic acid at room temperature for the period ranging from 16-24 hours, extracting with organic layer, washing with sodium metabisulphite, brine, drying and on evaporation to obtain 6-hydroxymethyl-4-(*tert*-butyldimethylsilyloxy)-(4*S*,6*R*)-tetrahydro-2*H*-2-pyranone having formula 1.

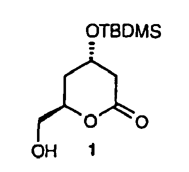

2. A process as claimed in claim 1, wherein the organic solvent used in steps a-c for the extraction of the product is selected from the group consisting of ethyl acetate, chloroform and dichloromethane.

3. A process as claimed in claim 1, wherein the buffer used in steps b for the reaction is selected from phosphate buffer and citrate buffer.

4. A process as claimed in claim 1, wherein the lipase used in steps b for the reaction is selected from the group consisting from pig procain lipase (PPL), pig liver esterase (PLE) and chicken liver acetone powder (CLAP).

5. A process as claimed in claim 1, wherein the enzymatic hydrolysis used in step b is mild and suppresses B-elimination along with enhancement in optical purity.

6. A process as claimed in claim 1, wherein the oxidising agent used is selected from the group consisting of chloroperbenzoic acid, hydrogen peroxide and peracetic acid.

7. A compound 3- oxo-5-(tert.butyldimethylsilyloxy-(1S, 5S)-cyclohexylacetate having formula 6:

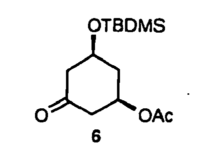

8. A compound 3-hydroxy-5(*tert*.butyldimethylsilyloxy)-(3*R*,5*S*)-cyclohexan-1 having formula 7

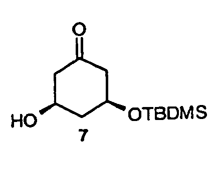

## Patentansprüche

1. Verfahren zur Herstellung von 6-Hydroxymethyl-4-(tert-butyldimethylsilyloxy)-(4S,6R)-tetrahydro-2H-2-pyranon der Formel 1, das umfasst:
a) Umsetzen von 3-Hydroxy-5-(tert-butyldimethyl-silyloxy)-(1S,3R,5R)-cyclohexylacetat der Formel 5 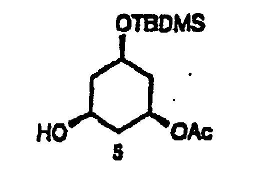 in einem organischen Lösemittel, vorzugsweise Chloralkanen, mit einem Chlorchromat von tertiären Aminen bei einer Temperatur im Bereich von 10 bis 30 °C, Quenchen der Reaktion durch Zugabe von Diethylether, Filtrieren des Gemischs über Ceilit und Waschen mit Kochsalzlösung, Entfernen des Lösemittels durch Abdampfen und anschließende Schnellsäulenfiltration, wobei 3-Oxo-5-(tert-butyldimethylsilyloxy)-(1S,5S)-cyclohexylacetat der Formel 6 erhalten wird, 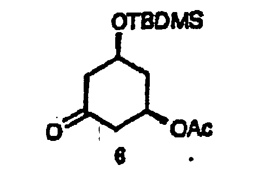
b) Hydrolysieren von 3-Oxo-5-(tert-butyldimethylsilyloxy)-(1S,5S)-cyclohexylacetat der Formel 6 mit einem Lipaseenzym in einem Puffer mit einem pH-Bereich von 5 bis 7 bei einer Temperatur im Bereich von 25 bis 30 °C über einen Zeitraum im Bereich zwischen 24 und 48 h, Extrahieren des Gemischs mit einem organischen Lösemittel, Entfernen des Lösemittels durch Abdampfen und Säulenchromatographie, wobei 3-Hydroxy-5-(tert-butyldimethylsilyloxy)-(3R,5S)-cyclohexan-1-on der Formel 7 erhalten wird, 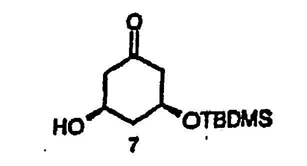
c) Umsetzen von 3-Hydroxy-5-(tert-butyldimethyl-silyloxy)-(3R,5S)-cyclohexan-1-on der Formel 7 mit m-Chlorperbenzoesäure bei Raumtemperatur über einen Zeitraum im Bereich von 16-24 h, Extrahieren mit einer organischen Schicht, Waschen mit Natriummetabisulfit, Kochsalzlösung, Trocknen und Eindampfen, wobei 6-Hydroxymethyl-4-(tert-butyldimethylsilyloxy)-(4S,6R)-tetrahydro-2H-2-pyranon der Formel 1 erhalten wird.

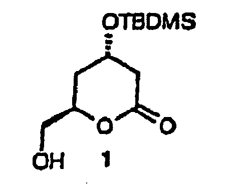

2. Verfahren gemäß Anspruch 1, wobei das in den Stufen a-c für die Extraktion des Produkts verwendete organische Lösemittel aus der Gruppe von Ethylacetat, Chloroform und Dichlormethan ausgewählt ist.

3. Verfahren gemäß Anspruch 1, wobei der in den Stufen b für die Umsetzung verwendete Puffer aus einem Phosphatpuffer und einem Citratpuffer ausgewählt ist.

4. Verfahren gemäß Anspruch 1, wobei die in den Stufen b für die Umsetzung verwendete Lipase aus der Gruppe von Pig Procain Lipase (PPL), Pig Liver Esterase (PLE) und Chicken Liver Acetone Powder (CLAP) ausgewählt ist.

5. Verfahren gemäß Anspruch 1, wobei die in Stufe b verwendete enzymatische Hydrolyse mild ist und eine β-Eliminierung unterdrückt wird und gleichzeitig die optische Reinheit verstärkt wird.

6. Verfahren gemäß Anspruch 1, wobei das verwendete Oxidationsmittel aus der Gruppe von Chlorperbenzoesäure, Wasserstoffperoxid und Peressigsäure ausgewählt ist.

7. Die Verbindung 3-Oxo-5-(tert-butyldimethylsilyloxy-(1S,5S)-cyclohexylacetat der Formel 6:

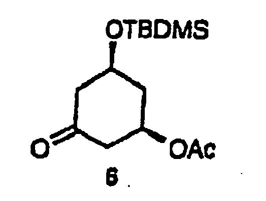

8. Die Verbindung 3-Hydroxy-5-(tert-butyldimethyl-silyloxy)-(3R,5S)-cyclohexan-1 der Formel 7

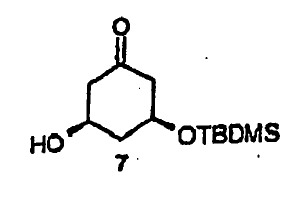

## Revendications

1. Procédé de préparation de la 6-hydroxyméthyl-4-(*tert*-butyldiméthylsilyloxy)-(4S,6R)-tétrahydro-2*H*-2-pyranone répondant à la formule 1 qui comprend les étapes suivantes :
a) faire réagir l'acétate de 3-hydroxy-5-(*tert-*butyldiméthylsilyloxy)-(1*S*,3*R*,5*R*)-cyclohexyle répondant à la formule 5 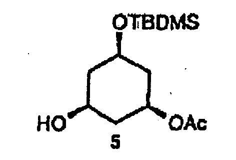 dans un solvant organique de préférence des chloroalcanes avec un chlorochromate d'amines tertiaires à une température située dans la gamme de 10 à 30 °C, arrêter la réaction en ajoutant de l'éther diéthylique, filtrer le mélange à travers de la Celite et laver avec de la saumure, éliminer le solvant par évaporation, suivi d'une filtration rapide sur colonne pour obtenir l'acétate de 3-oxo-5-(*tert-*butyldiméthylsilyloxy)-(1*S*,5*S*)-cyclohexyle répondant à la formule 6, 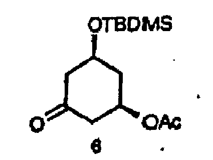
b) hydrolyser l'acétate de 3-oxo-5-(*tert-*butyldiméthylsilyloxy)-(1*S*,5*S*)-cyclohexyle répondant à la formule 6 avec une enzyme lipase dans un tampon ayant une gamme de pH de 5 à 7, à une température située dans la gamme de 25 à 30 °C pendant une durée dans la gamme située entre 24 à 48 heures, extraire le mélange avec un solvant organique, éliminer le solvant par évaporation et par chromatographie sur colonne pour obtenir la 3-hydroxy-5-(*tert*-butyldiméthylsilyloxy)-(3*R*,5*S*)-cyclohexan-1-one répondant à la formule 7, 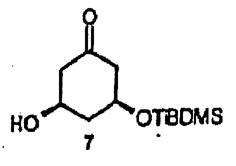
c) faire réagir la 3-hydroxy-5-(*tert-*butyldiméthylsilyloxy)-(3R,5S)-cyclohexan-1-one répondant à la formule 7 avec l'acide m-chloroperbenzoïque à la température ambiante pendant une durée située dans la gamme de 16 à 24 heures, extraire avec la phase organique, laver avec du métabisulfite de sodium, de la saumure, sécher et évaporer pour obtenir la 6-hydroxyméthyl-4-(*tert-*butyldiméthylsilyloxy)-(4*S*,6*R*)-tétrahydro-2*H*-2-pyranone répondant à la formule 1.

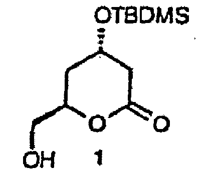

2. Procédé selon la revendication 1, dans lequel le solvant organique utilisé dans les étapes a à c pour l'extraction du produit est choisi dans le groupe constitué par l'acétate d'éthyle, le chloroforme et le dichlorométhane.

3. Procédé selon la revendication 1, dans lequel le tampon utilisé dans les étapes b pour la réaction est choisi parmi un tampon phosphate et un tampon citrate.

4. Procédé selon la revendication 1, dans lequel la lipase utilisée dans les étapes b pour la réaction est choisie dans le groupe constitué par la lipase de procaïne de porc (PPL), l'estérase de foie de porc (PLE) et la poudre acétonique de foie de poulet (CLAP).

5. Procédé selon la revendication 1, dans lequel l'hydrolyse enzymatique utilisée dans l'étape b est modérée et supprime la β-élimination tout en améliorant la pureté optique.

6. Procédé selon la revendication 1, dans lequel l'agent oxydant utilisé est choisi dans le groupe constitué par l'acide chloroperbenzoïque, le peroxyde d'hydrogène et l'acide acétique.

7. Composé acétate de 3-oxo-5-(tert-butyldiméthylsilyloxy)- (1*S*,5*S*)-cyclohexyle répondant à la formule 6 :

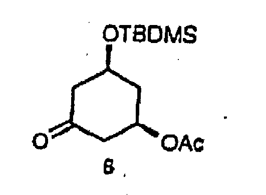

8. Composé 3-hydroxy-5-(tert-butyldiméthylsilyloxy)-(3*R*,5*S*)-cyclohexan-1-one répondant à la formule 7 :
